# EUROPÄISCHE PATENTSCHRIFT

(11) **EP 2 823 030 B1**
(45) Veröffentlichungstag und Bekanntmachung des Hinweises auf die Patenterteilung: **10.08.2022**
(21) Anmeldenummer: 13711314.8
(22) Anmeldetag: 28.02.2013
(51) Int. Cl.: C12M 1/00

(54) **VERFAHREN ZUR AUFARBEITUNG VON ROHGÜLLE**
METHOD FOR PROCESSING RAW LIQUID MANURE
PROCÉDÉ ET INSTALLATION POUR TRAITER DU PURIN BRUT

(30) Priorität: 06.03.2012 DE 102012004497
(43) Veröffentlichungstag der Anmeldung: 14.01.2015
(73) Patentinhaber: GEA Mechanical Equipment GmbH, 59302 Oelde (DE)
(72) Erfinder: HEYMANN, Sven, 06526 Sangerhausen (DE); LÜKING, Bernd, 33330 Gütersloh (DE)
(74) Vertreter: Specht, Peter
(86) Internationale Anmeldenummer: PCT/EP2013/053992
(87) Internationale Veröffentlichungsnummer: WO 2013/131798

(56) Entgegenhaltungen:
- EP-A1- 1 013 614
- WO-A1-2004/015120
- WO-A1-2005/105680
- WO-A1-2010/074953
- WO-A1-2011/038364
- DE-A1- 4 027 581
- DE-A1-102006 057 163
- US-A1- 2003 084 693
- US-A1- 2008 250 723

## Beschreibung

Die Erfindung betrifft ein Verfahren zur Aufarbeitung von Rohgülle.

Es ist bekannt, dass bei der Separation von Rohgülle und/oder Gärreste mit einer Vollmantel-Schneckenzentrifuge eine Feststoffphase und eine geklärte Flüssigphase anfallen. Während bei der Separation von Rohgülle die Feststoffphase beispielsweise zur Biogasgewinnung genutzt werden kann, wäre es wünschenswert, die Entsorgung der geklärten Flüssigkeitsphase weiter zu vereinfachen. Dies wäre - und hier setzt die Erfindung an - möglich, wenn die Flüssigkeitsphase noch weniger Trubstoffe bzw. Feinstpartikel enthalten würde und der CSB-Wert (chemischer Sauerstoffbedarf) der geklärten Flüssigkeitsphase gesenkt werden könnte. Bei diesem Gedanken setzt die Erfindung an, denn nach dem Stand der Technik ist eine solche Klärung bisher nicht oder nur mit sehr hohem Aufwand möglich.

Als nächstliegender Stand der Technik wird die DE 40 27 581 A1 genannt. Diese Druckschrift offenbart eine zentrifugale Grobreinigung von Gülle und eine anschließende Vorkonditionierung für eine weitergehende Bearbeitung der Flüssigphase in einer Flotationszelle. Konkret offenbart die DE 40 27 581, dass sich die Zugabe von Fe(III)-haltigen Flockungsmitteln und säurehaltigen Fällmitteln als vorteilhaft erweise. Durch die Flockungsmittel werden kolloidal-gelöste Inhaltsstoffe mit negativen Oberflächenladungen derart destabilisiert, dass sie sich zu größeren Flocken agglomerieren können. Die Zugabe eines Flockungshilfsmittels fördert das Wachstum der Mikroflocken zu leicht abtrennbaren Makroflocken. Dagegen werden durch die Zugabe von Fällmitteln, wie organischen Carbonsäuren oder anorganischen Mineralsäuren, die in der Gülle enthaltenen gelösten Eiweiße denaturiert. Hierdurch wird ihre Löslichkeit stark herabgesetzt, sie koagulieren spontan und bilden unlösliche Niederschläge, deren spezifisches Gewicht dem von Wasser ähnlich ist und die deshalb tendenziell aufschwimmen.

Eine weitere Druckschrift des Standes der Technik ist die WO2004/015120 A1 und die darin offenbarte Aufarbeitung einer Pflanzenbiomasse, Weichholzbiomasse, Hartholzbiomasse, Abwasserschlamm, Papiermühlenschlamm und der Biomassefraktion von kommunalen Festabfall. Weiterhin als Stand der Technik ist die WO 2011/038364 A1 zu nennen. Hierin wird ein Verfahren zur Herstellung einer 3-Hydroxypropionsäure beschrieben mit der Zwischenstufe eines zuckerhaltigen Fermentationsproduktes aus Stärke.

Es ist daher Aufgabe der vorliegenden Erfindung ein Verfahren zur Aufarbeitung von Rohgülle bereitzustellen, welches eine einfachere Entsorgung der geklärten Flüssigkeitsphase zulässt.

Die vorliegende Erfindung löst diese Aufgabe durch die Merkmale der Ansprüche 1.

Erfindungsgemäß umfasst ein Verfahren zur Aufarbeitung von Rohgülle folgende Schritte: Bereitstellen von Rohgülle; Klären der Rohgülle in einer ersten Klärstufe unter Bildung einer ersten Feststoffphase und einer ersten geklärten Flüssigkeitsphase; Vorkonditionieren der ersten geklärten Flüssigkeitsphase; und Klären der ersten geklärten sowie vorkonditionierten Flüssigkeitsphase in einer zweiten Klärstufe unter Bildung einer zweiten Feststoffphase und einer zweiten, weiter geklärten Flüssigkeitsphase.

Durch die infolge des Vorkonditionierens mögliche weitere Entfernung der Feinstpartikel bzw. Trubstoffe wird der CSB-Gehalt der zweiten geklärten Flüssigkeitsphase gegenüber der ersten geklärten Flüssigkeitsphase soweit gesenkt, dass ein Einleiten in eine biologische Stufe eines Klärwerkes möglich wird. Der CSB-Wert (chemischer Sauerstoffbedarf insbesondere in mg/l) ist ein Maß für die Summe aller organischen Verbindungen in Wasser, einschließlich der schwer abbaubaren Verbindungen.

Das Vorkonditionieren kann dabei im ersten Schritt erfindungsgemäß und einfach durch ein Zudosieren eines Flockungsmittels und Änderung des pH-Wertes erfolgen. Es dient der Bildung von größeren Feststoffpartikeln durch Flockung.

Dabei ist es beim Zudosieren des Flockungsmittels von Vorteil, wenn im zweiten Schritt ein zusätzliches Zudosieren eines Flockungshilfsmittels zur Verbesserung der Scherfestigkeit eines Koagulates erfolgt. Durch die bessere Scherfestigkeit kann das Koagulat auch höheren Zentrifugalkräften ausgesetzt werden und besser aus der ersten (vor-)geklärten Flüssigphase durch einen weiteren Klärvorgang abgetrennt werden.

Erfindungsgemäß erfolgt das Klären der Rohgülle in der ersten Klärstufe in Schritt ii) durch eine Zentrifuge, insbesondere eine Vollmantelschneckenzentrifuge. Es kann zwar auch eine Pressschnecke zur Entwässerung der Rohgülle genutzt werden. Es hat sich allerdings gezeigt, dass Schleimstoffe sich besonders effektiv durch den Einsatz einer Zentrifuge aus der Klarphase entfernen lassen, während bei Einsatz einer Pressschnecke diese Schleimstoffe zum überwiegenden Teil in der ersten geklärten Flüssigkeitsphase verbleiben und deren Klärung in der zweiten Klärstufe erschweren.

Um die biologische Abbaubarkeit der zweiten Feststoffphase zu erleichtern, ist es von Vorteil, wenn das Flockungshilfsmittel ein Polymer auf Wasserbasis ist.

Die in Schritt ii) gewonnene erste Feststoffphase kann z.B. zur Gewinnung von Biogas verwendet werden oder als phosphatreicher Dünger.

Die zweite geklärte Flüssigkeitsphase weist relativ zur ersten geklärten Flüssigkeitsphase vorteilhaft einen verringerten CSB-Wert auf. Ein geringer CSB-Wert einer Flüssigkeit resultiert in einer geringeren Belastung bei deren Einleitung in ein Klärwerk. Die zweite Feststoffphase kann ebenfalls vielfältig genutzt werden, so wiederum zur Biogasgewinnung oder als phosphatreicher Dünger.

Vorteilhafte Ausgestaltungen der Erfindung sind Gegenstand der Unteransprüche.

Eine Anlage zur Aufarbeitung von Rohgülle umfasst zumindest eine erste Zentrifuge, insbesondere eine Vollmantelschneckenzentrifuge, zur Entwässerung von Rohgülle unter Bildung einer ersten geklärten Flüssigkeitsphase und einer ersten Feststoffphase und eine erste Dosieranlage zur Zugabe eines Flockungsmittels zur ersten geklärten Flüssigkeitsphase oder eines Mittels zur Änderung des pH-Wertes der ersten geklärten Flüssigkeitsphase.

Die Zugabe eines Flockungsmittels zur Rohgülle ist bereits bekannt. Allerdings erhöht eine derartige Zugabe die chemische Belastung der ersten Feststoffphase. Außerdem befindet sich trotz der Zugabe eines Flockungsmittels ein erheblicher Anteil an Feinstpartikeln noch in der ersten geklärten Flüssigkeitsphase. Die Zugabe des Flockungsmittels oder eines Mittels zur Änderung des pH-Wertes zur ersten geklärten Flüssigkeitsphase, also erst nach der ersten Klärstufe, ermöglicht hingegen die Gewinnung einer weitestgehend feinstpartikelfreien Klarphase durch mechanische Klärung in einer zweiten Klärstufe.

Vorteilhafte Ausgestaltungen der Erfindung sind der Gegenstand der übrigen Unteransprüche.

Nachfolgend wird ein bevorzugtes Ausführungsbeispiel anhand der Zeichnung näher erörtert. Es zeigt:

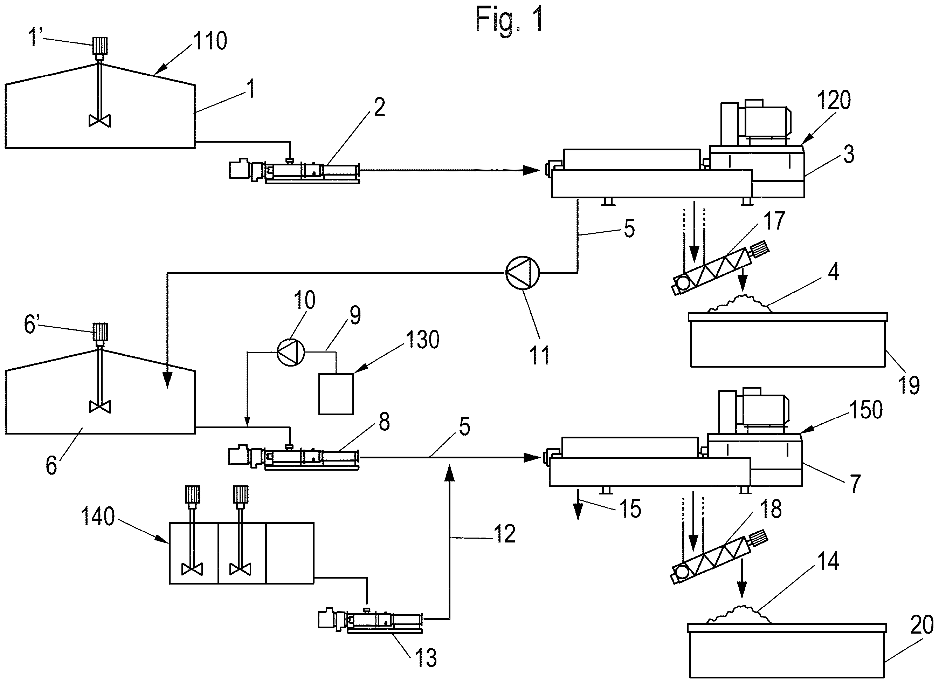

Fig.1 eine schematische Darstellung eines Verfahrens zur Aufarbeitung von Rohgülle.

Nach dem erfindungsgemäßen Verfahren wird Rohgülle (die z.B. einen CSB Wert um 40000 aufweist) zunächst in einem ersten Tank 1 gesammelt. Um eine möglichst gleichmäßige Viskosität zu erhalten und um ein Absetzen von Feststoffen nach Möglichkeit zu vermeiden, weist der Tank 1 vorzugsweise ein Rührwerk 1' auf.

Von dem ersten Tank 1 wird die gesammelte Rohgülle hier über eine Förderpumpe 2 in eine erste Zentrifuge 3, vorzugsweise eine Vollmantelschneckenzentrifuge, geleitet. In dieser ersten Zentrifuge 3 erfolgt in einer ersten Klärstufe 120 eine Klärung in eine erste Feststoffphase 4 und eine erste geklärte Flüssigkeitsphase 5.

Die erste Feststoffphase wird hier beispielhaft über eine Förderschnecke 17 in einen Sammelbehälter 19 geleitet.

Die erste geklärte Flüssigkeitsphase 5 wird über eine Pumpe 11 in einem zweiten Tank 6 geleitet und dort gesammelt. Auch dieser zweite Tank 6 weist vorzugsweise ein Rührwerk 6' auf, um eine möglichst einheitliche Viskosität der gesammelten ersten geklärten Flüssigkeitsphase 5 zu erreichen.

Von dem zweiten Tank 6 wird die erste geklärte Flüssigkeitsphase 5 mittels einer dritten Förderpumpe 8 in eine zweite Zentrifuge 7 gepumpt.

Es ist vorteilhaft, wenn der ersten geklärte Flüssigkeitsphase in der Vorkonditionierung das Flockungsmittel 9 zugesetzt wird. Als das Flockungsmittel eignen sich insbesondere PAC (Polyaluminiumchlorid) oder FeCl₃ (Eisen(III)Chlorid).

Hierzu wird eine Dosiermenge des Flockungsmittels 9 beispielsweise aus einem Vorratstank mittels einer Dosiereinrichtung (beispielsweise einer Pumpe 13) zudosiert. Die Vorkonditionierung kann während des Überführens der ersten geklärten Flüssigkeitsphase 5 von der ersten Zentrifuge 3 in den zweiten Tank 6 erfolgen und/oder direkt im zweiten Tank 6 und/oder nach dem zweiten Tank vor oder spätestens bei dem nochmaligen Einleiten in eine Kläreinrichtung wie eine weitere Zentrifuge (was weiter unten noch näher erläutert wird).

Durch die Zugabe von Flockungsmittel 9 erfolgt nach einer gewissen Einwirkzeit ein Koagulieren von Feinstbestandteilen in der Klarphase zu größeren Makroflocken.

Es ist besonders von Vorteil, wenn zusätzlich zur Zugabe eines Flockungsmittels 9 auch die Zugabe eines Flockungshilfsmittels 12 erfolgt, um die Scherstabilität des Koagulates bzw. der gebildeten Makroflocken zu erhöhen. Bevorzugt aber nicht zwingend handelt es sich bei dem Flockungshilfsmittel 12 um eine mineralölfreie Chemikalie, besonders bevorzugt um ein Polymer auf Wasserbasis, um die Umweltverträglichkeit nicht zu beeinträchtigen.

In dem in Fig. 1 dargestellten Ausführungsbeispiel erfolgt die Zugabe des Flockungshilfsmittels 12 über eine dritte Förderpumpe 13 nach dem zweiten Tank 6.

Anschließend wird die erste geklärte Flüssigkeitsphase 5 mit den koagulierten Feinstbestandteilen direkt oder nach einem gewissen Zeitraum in eine zweite Zentrifuge 7 eingeleitet. In dieser zweiten Zentrifuge 7 - die erfindungsgemäß als eine Vollmantel-Schneckenzentrifuge ausgebildet ist - erfolgt eine weitere Klärung der ersten "vor"geklärten Flüssigkeitsphase 5 unter Ausbildung einer zweiten geklärten Flüssigkeitsphase 15 - die auch als Klarphase bezeichnet werden kann - und einer zweiten Feststoffphase 14.

Die zweite Feststoffphase 14 wird hier beispielhaft über eine (oder dieselbe) Förderschnecke 17 oder 18 in einen (oder denselben) Sammelbehälter 19 oder 20 geleitet.

Nachfolgend werden anhand des Ausführungsbeispiels der Fig.1 einzelne Verfahrensschritte in vorteilhafter Ausgestaltung detaillierter erläutert.

Im ersten Tank 1 erfolgt ein Bereitstellen von Rohgülle 110. Dieses Bereitstellen kann beispielsweise durch Sammeln von Rohgülle in dem Tank 1 erfolgen.

Nach dem Bereitstellen von Gülle 110 erfolgt ein Klären der Rohgülle in einer ersten Klärstufe 120. Dabei wird die Rohgülle mittels der ersten Zentrifuge 5 unter Bildung einer ersten geklärten Flüssigkeitsphase 5 und einer ersten Feststoffphase 6 geklärt.

Dieses Klären der Rohgülle erfolgt vorzugsweise ohne chemische Zusätze. Der Feststoff wird in dieser ersten Klärstufe 120 nicht maximal entwässert. Das bewirkt eine Abtrennung von Schleimstoffe, überwiegend Phosphate, mit dem Feststoff. Man erhält somit einen phosphatreichen Feststoff, welcher entsorgt oder aufgrund der hohen Nährstoff-Konzentration ggf. auch als Dünger verwendet werden kann. Alternativ kann dieser Feststoff aufgrund seines hohen Biogaspotentials auch als Ausgangsmaterial in Biogasanlagen eingesetzt werden.

Bei der ersten Klärstufe 120 kann sowohl eine Pressschnecke als auch eine Zentrifuge, insbesondere eine Vollmantelschneckenzentrifuge, vorzugsweise ein Dekanter, eingesetzt werden, dessen Verwendung jedoch bevorzugt gegenüber der Verwendung einer Pressschnecke ist.

Denn es hat sich gezeigt, dass die Abtrennung der phosphathaltigen Schleimstoffe, bei Verwendung von Pressschnecken nur in geringem Umfang erfolgt, da bei Pressschnecken die Rohgülle gefiltert wird/werden, wodurch lediglich grobe Bestandteile aus der Klarphase entfernt werden.

Demgegenüber werden die phosphathaltigen Schleimstoffe beim zentrifugalen Klären in einem Dekanter nahezu vollständig aus der Klarphase entfernt. Aufgrund der Reduzierung an Schleimstoffen wird zugleich die Viskosität der Klarphase gegenüber der Rohgülle vorteilhaft gesenkt, so dass die erste geklärte Flüssigkeitsphase 5 nach der ersten Klärstufe 120 annähernd die Viskosität von Wasser aufweist. Bereits die erste geklärte Flüssigkeitsphase 5 enthält lediglich noch Feinstpartikel und Trubstoffe, weist einen niedrigen Phosphatwert auf und hat die Viskosität von Wasser.

An das Klären der Rohgülle in einer ersten Klärstufe 120 schließt sich das Vorkonditionieren der ersten geklärten Flüssigkeitsphase 5 zur Klärung weiterer Feinstpartikeln aus ersten geklärten Flüssigkeitsphase 5 an.

Das Vorkonditionieren erfolgt erfindungsgemäß durch das Zudosieren 130 des Flockungsmittels 9 unter Bildung eines Koagulates. Zur weiteren Optimierung des Koagulats kann zusätzlich noch das Zudosieren 140 des Flockungshilfsmittels erfolgen, wodurch die gebildeten Makroflocken eine höhere Scherstabilität erhalten.

Erfindungsgemäß erfolgt das Vorkonditionieren der ersten geklärten Flüssigkeitsphase 5 zur Abtrennung von Feinstpartikeln aus der ersten geklärten Flüssigkeitsphase 5 durch ein, vorzugsweise mehrstufiges, pH-Wert-Verschieben. Dabei wird der pH-Wert der ersten geklärten Flüssigphase in den sauren Bereich, erfindungsgemäß auf einen pH-Wert ≤ 5,8, besonders bevorzugt ≤ 5,5 eingestellt. In diesem leicht sauren Bereich erfolgt die Ausbildung des Koagulats in Form von Hydroxidflocken offenbar aufgrund einer Ladungsumkehr am isoelektrischen Punkt. Besonders bevorzugt ist dabei ein pH-Wertbereich größer als pH = 3,8.

Im Anschluss an das Vorkonditionieren der ersten geklärten Flüssigkeitsphase 5 zur Klärung von Feinstpartikeln aus ersten geklärten Flüssigkeitsphase 5 erfolgt das bereits erwähnte Klären der ersten geklärten Flüssigkeitsphase 5 in der zweiten Klärstufe 150 unter Bildung der zweiten geklärten Flüssigkeitsphase 15 und der Feststoffphase 14.

Sofern das Vorkonditionieren der ersten geklärten Flüssigkeitsphase 5 durch pH-Wertverschiebung erfolgt, so wird erfindungsgemäß nach der Klärung in der zweiten Klärstufe 150 der pH-Wert wieder auf einen Wert zwischen 6,5 und 7,5 angehoben. Die gebildete zweite geklärte Flüssigkeitsphase 15 weist sehr gute Voraussetzungen für eine biologische Stufe oder Umkehrosmose auf. So kann der CSB Wert gegenüber der Phase 5 nochmals deutlich weiter auf bis ca. 4000 (bei der Verarbeitung von Rohgülle). Sie kann ferner beispielsweise verregnet werden oder über Schleppschläuche ausgebracht werden, so dass der in der Flüssigkeit gelöste Stickstoff besonders gut direkt den Wurzeln von Pflanzen weitgehend ohne Stickstoffverluste zur Verfügung gestellt wird.

Die zweite geklärte Feststoffphase 5 weist relativ geringe TS-Werte auf (Trockensubstanzwerte), da durch die vorherige Abtrennung von groben Partikeln kein Stützgerüst mehr im restlichen Feststoff vorhanden ist. Insbesondere weist aber die zweite geklärte Flüssigkeitsphase 15 relativ zur ersten geklärten Flüssigkeitsphase 5 vorteilhaft einen verringerten CSB-Wert auf. Sie kann verschieden verarbeitet werden, so kann sie mit den phosphatreichen Phase 4 gemischt werden und mit dieser gemeinsam genutzt oder entsorgt werden.

**Bezugszeichenliste**

| | | |
|---|---|---|
| Tank | 1 | |
| Rührwerk | 1 ' | |
| Förderpumpe | 2 | |
| Zentrifuge | 3 | |
| Feststoffphase | 4 | |
| Flüssigkeitshase | 5 | |
| Tank | 6 | |
| Rührwerk | 6' | |
| Zentrifuge | 7 | |
| Förderpumpe | 8 | |
| Flockungsmittel | 9 | |
| Pumpe | 10 | |
| Pumpe | 11 | |
| Flockungshilfsmittel | 12 | |
| Pumpe | 13 | |
| Feststoffphase | 14 | |
| Klarphase | 15 | |
| Förderschnecke | 17 | |
| Förderschnecke | 18 | |
| Sammelbehälter | 19 | |
| Sammelbehälter | 20 | |
| Bereitstellen von Rohgülle | | 110 |
| erste Klärstufe | | 120 |
| Zugeben eines Flockungsmittels | | 130 |
| Zugeben eines Flockungshilfsmittels | | 140 |
| zweite Klärstufe | | 150 |

## Patentansprüche

1. Verfahren zur Aufarbeitung von Rohgülle, **gekennzeichnet durch** folgende Schritte:
i) Bereitstellen von Rohgülle (110);
ii) Klären der Rohgülle in einer ersten Klärstufe (120) unter Bildung einer ersten Feststoffphase (4) und einer ersten geklärten Flüssigkeitsphase (5);
wobei das Klären der Rohgülle in der ersten Klärstufe (120) in Schritt ii) durch eine erste Zentrifuge (3) erfolgt
iii) Vorkonditionieren der ersten geklärten Flüssigkeitsphase (5), wobei das Vorkonditionieren durch Zugeben eines Flockungsmittels (130) und durch ein Verschieben des pH-Wertes, wobei das Verschieben des pH-Wertes in den sauren Bereich auf einen pH-Wert ≤ 5,8 erfolgt; und
iv) Klären der ersten geklärten sowie vorkonditionierten Flüssigkeitsphase (5) in einer zweiten Klärstufe (150) unter Bildung einer zweiten Feststoffphase (14) und einer zweiten, weiter geklärten Flüssigkeitsphase (15) bzw. Klarphase,
wobei nach dem Klären der ersten geklärten sowie vorkonditionierten Flüssigkeitsphase (5) in der zweiten Klärstufe (150), gemäß Schritt iv), die zweite, weiter geklärte Flüssigkeitsphase auf einen pH-Wert ≥ 6,5 eingestellt wird
wobei das Klären der ersten geklärten Flüssigkeitsphase (5) in der zweiten Klärstufe (150) durch eine zweite Zentrifuge (7) als Vollmantelschneckenzentrifuge erfolgt.

2. Verfahren nach Anspruch 1, **dadurch gekennzeichnet, dass** das Klären der Rohgülle in der ersten Klärstufe (120) in Schritt ii) durch eine Vollmantelschneckenzentrifuge erfolgt.

3. Verfahren nach Anspruch 2, **dadurch gekennzeichnet, dass** das Verschieben des pH-Wertes in den sauren Bereich auf einen pH-Wert ≤ 5,5 erfolgt.

4. Verfahren nach einem der vorhergehenden Ansprüche, **dadurch gekennzeichnet, dass** das Verschieben des pH-Wertes in den sauren Bereich auf einen pH-Wert > 3,8 erfolgt.

5. Verfahren nach Anspruch 3, **dadurch gekennzeichnet, dass** nach dem Klären der ersten geklärten sowie vorkonditionierten Flüssigkeitsphase (5) in der zweiten Klärstufe (150), gemäß Schritt iv), die zweite, weiter geklärte Flüssigkeitsphase in einen pH-Wertbereich zwischen 6,5 und 7,5 eingestellt wird.

6. Verfahren nach Anspruch 4, **dadurch gekennzeichnet, dass** zusätzlich zum Zugeben eines Flockungsmittels (130) ein Zugeben eines Flockungshilfsmittels (140) zur Verbesserung der Scherfestigkeit eines Koagulates erfolgt.

7. Verfahren nach einem der vorhergehenden Ansprüche, **dadurch gekennzeichnet, dass** das Flockungsmittel (9) PAC oder FeCl₃ ist.

8. Verfahren nach einem der vorhergehenden Ansprüche, **dadurch gekennzeichnet, dass** das Flockungshilfsmittel (12) ein Polymer auf Wasserbasis ist.

9. Verfahren nach einem der vorhergehenden Ansprüche, **dadurch gekennzeichnet, dass** die zweite weiter geklärte Flüssigkeitsphase einer biologischen Stufe oder einer Umkehrosmose zugeführt wird.

## Claims

1. A method for processing raw liquid manure, **characterized by** the following steps:
i) provision of raw liquid manure (110);
ii) treatment of the raw liquid manure in a first treatment stage (120) with formation of a first solid phase (4) and a first treated liquid phase (5); wherein the treatment of the raw liquid manure in the first treatment stage (120) in step ii) is carried out by a first centrifuge (3).
iii) precondition of the first treated liquid phase (5); wherein said preconditioning is performed by adding a flocculant (130) and by shifting the pH, wherein said shifting of the pH into the acidic range is performed to a pH value of ≤ 5.8 and
iv) treatment of the first treated and preconditioned liquid phase (5) in a second treatment stage (150) with formation of a second solid phase (14) and a second, further treated liquid phase (15) or clarified phase,
wherein after clarification of the first clarified and preconditioned liquid phase (5) in the second treatment stage (150), according to step iv), the second, further clarified liquid phase is adjusted to a pH value of ≥ 6.5
wherein the treatment of the first treated liquid phase (5) in the second treatment stage (150) is effected by a second centrifuge (7) as solid bowl scroll centrifuge.

2. The method as claimed in claim 1, **characterized in that** the treatment of raw liquid manure in the first treatment stage (120) is effected in step ii) by a solid bowl scroll centrifuge.

3. The method as claimed in claim 2, **characterized in that** the shifting of the pH takes place into the acidic range to a pH ≤ 5.5.

4. The method as claimed in one of the previous claims, **characterized in that** the shifting the pH value into the acidic range to a pH value of > 3.8.

5. The method as claimed in claim 3, **characterized in that** after the treatment of the first treated and preconditioned liquid phase (5) in the second treatment stage (150), according to step iv), the second, further treated liquid phase is adjusted to a pH between 6.5 and 7.5.

6. The method as claimed in claim 4, **characterized in that** in addition to the addition of a flocculant (130), an addition of a flocculation aid (140) is effected to improve the shear strength of a coagulate.

7. The method as claimed in one of the previous claims, **characterized in that** the flocculant (9) is PAC or FeCl₃.

8. The method as claimed in one of the previous claims, **characterized in that** the flocculation aid (12) is a water-based polymer.

9. The method as claimed in one of the previous claims, **characterized in that** the second further treated liquid phase is fed to a biological or a reverse osmosis system.

## Revendications

1. Procédé pour le traitement de lisier brut, **caractérisé en ce qu'**il comprend les étapes suivantes :
i) apport de lisier brut (110) ;
ii) épuration du lisier brut dans une première étape d'épuration (120) avec formation d'une première phase solide (4) et d'une première phase liquide épurée (5) ;
l'épuration du lisier brut étant réalisée, dans la première étape d'épuration (120) de l'étape ii), par une première centrifugeuse (3) ;
iii) préconditionnement de la première phase liquide épurée (5), le préconditionnement consistant à ajouter un agent de floculation (130) et à décaler le pH, le décalage du pH étant réalisé dans la plage acide jusqu'à une valeur de pH de ≤ 5,8 ; et
iv) épuration de la première phase liquide (5) épurée et préconditionnée dans une deuxième étape d'épuration (150) avec formation d'une deuxième phase solide (14) et d'une deuxième phase liquide (15) encore plus épurée ou phase claire,
le pH de la deuxième phase liquide encore plus épurée étant ajusté à ≥ 6,5 après l'épuration de la première phase liquide (5) épurée et préconditionnée dans la deuxième étape d'épuration (150), selon l'étape iv),
l'épuration de la première phase liquide épurée (5) étant effectuée dans la deuxième étape d'épuration (150) par une deuxième centrifugeuse (7) qui est une centrifugeuse à vis à bol plein.

2. Procédé selon la revendication 1, **caractérisé en ce que** l'épuration du lisier brut dans la première étape d'épuration (120) est effectuée dans l'étape ii) par une centrifugeuse à vis à bol plein.

3. Procédé selon la revendication 2, **caractérisé en ce que** le pH est décalé dans la plage acide jusqu'à une valeur de pH de ≤ 5,5.

4. Procédé selon l'une des revendications précédentes, **caractérisé en ce que** le pH est décalé dans la plage acide jusqu'à une valeur de pH de > 3,8.

5. Procédé selon la revendication 3, **caractérisé en ce qu'**après l'épuration dans l'étape iv) de la première phase liquide (5) épurée et préconditionnée dans la deuxième étape d'épuration (150), le pH de la deuxième phase liquide encore plus épurée est ajusté dans une plage de pH de 6,5 à 7,5.

6. Procédé selon la revendication 4, **caractérisé en ce qu'**outre l'ajout d'un agent de floculation (130), un adjuvant de floculation (140) est ajouté afin d'améliorer la résistance au cisaillement d'un coagulat.

7. Procédé selon l'une des revendications précédentes, **caractérisé en ce que** l'agent de floculation (9) est du PAC ou du FeCl₃.

8. Procédé selon l'une des revendications précédentes, **caractérisé en ce que** l'adjuvant de floculation (12) est un polymère à base aqueuse.

9. Procédé selon l'une des revendications précédentes, **caractérisé en ce que** la deuxième phase liquide encore plus épurée est amenée à une étape de traitement biologique ou à une osmose inverse.
